# EUROPEAN PATENT APPLICATION

(11) **EP 4 620 434 A2**
(43) Date of publication of application: **24.09.2025**
(21) Application number: 25186583.8
(22) Date of filing: 07.10.2021
(51) Int. Cl.: A61F 2/24

(54) **DEVICE FOR TREATING VALVULAR INSUFFICIENCY**

(30) Priority: 09.10.2020 US 202063089587 P
(62) Divisional of application: 21877142.6
(71) Applicant: Coramaze Technologies Ltd., Petach-Tikva 4952801 (IL)
(72) Inventor: HARARI, Boaz, 5592535 Ganei Tikva (IL); SADAN, Ido, 6813936 Tel-Aviv (IL); GERHARDT, Thomas, 7806 Cape Town (ZA)
(74) Representative: Sonnenberg Harrison Partnerschaft mbB

(57) **Abstract**

A device for treating valvular regurgitation that includes an expandable anchor designed for transitioning from a linear configuration to a crown-like configuration through a radial expansion phase and an inversion phase. The expandable anchor is attached to a transvalvular spacer for providing a coaptation surface to valve leaflets.

## Description

### RELATED APPLICATION/S

This application claims the benefit of priority of U.S. Provisional Patent Application No. 63/089,587 filed on 9 October 2020, the contents of which are incorporated herein by reference in their entirety.

### FIELD AND BACKGROUND OF THE INVENTION

The present invention relates to a device for treating valvular insufficiency and to a catheter system for delivering the device into a heart. Embodiments of the present invention relate to a device that includes a trans-valvular spacer attached to a non-traumatic anchor positionable within the atrium.

Valvular insufficiency is a cardiac disease characterized by the failure of a cardiac valve to fully close leading to valvular regurgitation or leakage.

Anatomically, the valves are part of the dense connective tissue of the heart known as the cardiac skeleton and are responsible for the regulation of blood flow through the heart and great vessels. Valvular insufficiency due to failure or dysfunction can result in diminished heart functionality and a decrease in blood flow through the body. Treatment of damaged valves may involve medication alone, surgical valve repair (valvuloplasty) or replacement (insertion of an artificial heart valve).

Atrioventricular valvular insufficiency can lead to blood leakage or flow from the ventricle back into the atrium (regurgitation), rather than being forced out of the ventricle upon contraction.

Mitral regurgitation is a common valvular insufficiency and is typically treated via mitral valve replacement or mitral valve repair through open heart surgery or a minimally invasive (percutaneous) procedure. Percutaneous procedures for mitral valve repair include percutaneous mitral valve replacement, enhanced mitral coaptation, edge-to-edge-percutaneous mitral valve repair (plication), percutaneous chordal repair, percutaneous mitral annuloplasty, and left ventricle remolding.

While percutaneous mitral valve replacement/repair is less traumatic to the patient and can be used in patient populations that are not candidates for open heart surgery (due to age or co-morbidities), there are still challenges with delivering, and positioning the implant and with the stability and function of the implant in the heart over extended time periods.

US 2019/083238 A1 describes a heart implant comprising a tubular attachment element for attaching a sheath.

US 2013/046376 A1 describes a method of reducing the risk of clinical sequelae to catheter induced vascular injuries may include introducing a guide wire into a vascular sheath residing in a blood vessel.

US 2019/247190 A1 describes a heart implant comprising an attachment element, particularly a tubular attachment element for attaching a sheath.

WO 2020/197854 A1 describes devices for anchoring catheter systems, such as an atrioventricular heart valve regurgitation reduction system with a coapting element or spacer positioned within the atrioventricular valve leaflets.

There is thus a need for, and it would be highly advantageous to have, a device for correcting valvular insufficiency and a system for delivering same devoid of the limitations of presently used approaches.

### SUMMARY OF THE INVENTION

According to one aspect of the present invention there is provided a device for treating valvular regurgitation comprising an expandable anchor including a plurality of struts designed for transitioning from a linear configuration when trapped within a delivery tube to a crown-like configuration when released from the tube, the transitioning includes a radial expansion phase in which ends of the struts curl out away from a center line of the crown-like configuration and an inversion phase in which ends of the struts curl towards the center line of the crown-like configuration; and a transvalvular spacer attached to the expandable anchor configured for providing a coaptation surface to valve leaflets.

According to embodiments of the present invention the transvalvular spacer includes a flexible column enclosed in an inflatable balloon.

According to embodiments of the present invention the flexible column is laminated with a polymer sheath.

According to embodiments of the present invention the balloon is bonded to the polymer sheath.

According to embodiments of the present invention the flexible column is a tube having cutouts.

According to embodiments of the present invention the plurality of struts are attached around a proximal end portion of the flexible column.

According to embodiments of the present invention the polymer sheath includes an opening covered by a tube valve.

According to embodiments of the present invention the balloon is fillable with a fluid through the tube valve.

According to embodiments of the present invention the balloon is fillable with the fluid when the flexible column is mounted over a guidewire.

According to embodiments of the present invention a distal end of the flexible column includes a seal for sealing around the guidewire.

According to embodiments of the present invention the flexible column includes a tab for guiding an actuating mechanism to the tube valve.

According to embodiments of the present invention the expandable anchor includes seven struts.

According to embodiments of the present invention when the expandable anchor is expanded, each of the seven struts branches and rejoins along a length of each strut.

According to embodiments of the present invention adjacent struts of the seven struts are interconnected at a region between branching and rejoining.

According to embodiments of the present invention the ends of the struts are spoon-like in shape.

According to embodiments of the present invention the ends of the struts include an eyelet.

According to embodiments of the present invention a portion of the flexible column interposed between the expandable anchor and the transvalvular spacer includes a spiral cutout.

According to embodiments of the present invention the transvalvular spacer is detachably attached to the expandable anchor.

According to embodiments of the present invention at least one end of the inflatable balloon is inverted inward.

According to embodiments of the present invention the inflatable balloon includes a solution having an osmotic potential that is greater than that of blood.

According to embodiments of the present invention the expandable anchor includes a graspable element at a proximal end.

According to embodiments of the present invention the graspable element is a ball-shaped element.

According to another aspect of the present invention there is provided method of treating valvular regurgitation comprising: (a) delivering a device into a heart, the device including: (i) an expandable anchor including a plurality of struts designed for transitioning from a linear configuration when trapped within a delivery tube to a crown-like configuration when released from the tube, the transitioning includes a radial expansion phase in which ends of the struts curl out away from a center of the crown-like configuration and an inversion phase in which ends of the struts curl towards a center of the crown-like configuration; and (ii) a transvalvular spacer attached to the expandable anchor and including a flexible column enclosed in an inflatable balloon designed for providing a coaptation surface to valve leaflets; (b) releasing the expandable anchor in an atrium such that transvalvular spacer is positioned within a valve; and (c) at least partially inflating the inflatable balloon.

According to embodiments of the present invention (c) is effected using a solution having an osmotic potential that is greater than that of blood.

According to embodiments of the present invention step (c) precedes step (b).

According to another aspect of the present invention there is provided catheter system for delivering a device for treating valvular regurgitation comprising a co-axial catheter assembly including: (a) a first catheter including a distal grasper for grasping the device; (b) a second catheter movable along the first catheter and having a distal locking cup for locking the grasper therein; and (c) a third catheter movable along the second catheter.

According to embodiments of the present invention the grasper includes an O-ring for sealing against the device.

According to embodiments of the present invention the catheter assembly includes at least one fluid conduit for providing a fluid to the device when attached thereto.

According to embodiments of the present invention the system further comprises an elongated element movable out of a distal end of the co-axial catheter assembly, the elongated element being for opening a valve of the device to allow fluid delivery thereto from the at least one fluid conduit.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE SEVERAL VIEWS OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 illustrates an embodiment of anchor portion of the present device.
FIG. 2 illustrates an embodiment of the present device including the anchor portion and attached spacer portion.
FIGs. 3A-D illustrate expansion of the anchor portion when pushed out of a constraining catheter tube.
FIG. 4 illustrates an embodiment of the present device showing the internal components of the spacer portion.
FIG. 5 illustrates the balloon of the spacer portion showing an inverted distal end of the balloon.
FIG. 6 illustrates the bent tab of the center column of the spacer portion that directs access to the balloon-filling valve.
FIGs. 7A-B illustrate an embodiment of a catheter system for delivering the present device into a heart.
FIG. 8 and FIG. 9 illustrate the distal end of the catheter system of Figures 7A-B disengaged from (Figure 8) and engaged to (Figure 9) the proximal end of the device of the present invention.
FIGs. 10A-B illustrate the present device positioned in a heart at systole (Figure 10A) and diastole (Figure 10B) phases of the heart.

### DESCRIPTION OF SPECIFIC EMBODIEMENTS OF THE INVENTION

The present invention is of a device which can be used to correct valvular insufficiency. Specifically, the present invention can be used to percutaneously treat valvular insufficiencies by delivering, positioning and anchoring a device that includes a trans-valvular spacer attached to a non-traumatic atrial anchor.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Implants and approaches for correcting valvular insufficiencies that result from incomplete leaflet coaptation have been described in the prior art. Such devices include atrial or ventricular anchors attached to spacers that are positioned within the valve opening to seal against the native valve leaflets when closed. Such implants are generally effective in sealing against the closed leaflets but are oftentimes delivered or anchored in a manner that is traumatic to heart tissue.

While reducing the present invention to practice, the present inventors have devised an implant and delivery system that address these limitations of prior art devices while providing numerous additional benefits in function and long term stability.

The present invention is characterized by:
(i) an expandable atrial anchor that applies an outward radial force to anchor against the inner walls of the atrium and as such is non-traumatic to atrial tissue and stable over numerous heart cycles;
(ii) an expansion mechanism that maintains any potential traumatic ends of the expandable atrial anchor away from the atrial inner walls;
(iii) a leaflet-coaptation spacer that can be individually sized (diameter) according to patient needs prior to or following delivery and at any point during the procedure;
(iv) a leaflet-coaptation spacer that can self-size (diameter) according to patient needs following delivery;
(v) a delivery system that firmly grasps the implant throughout expansion of the expandable atrial anchor and allows the expandable atrial anchor to self-position in the most optimal orientation in the atrium;
(vi) an atraumatic expandable anchor that can self-reposition within the anatomy;
(vii) an expandable anchor that allows for assessment of in-situ efficacy within the anatomy while maintaining full retrievability;
(viii) a delivery system that allows for implant positioning, filling, evacuation and retrieval

Thus, according to one aspect of the present invention there is provided a device (implant) for treating valvular insufficiency. As used herein, the phrase "valvular insufficiency" relates to any valve leaflet dysfunction that leads to valvular leakage and regurgitation. Valvular insufficiency can be caused by, for example, congenital heart disease, infection, leaflet stenosis, widening or stretching of the valve annulus, chordae rupture, dilated cardiomyopathy or valve prolapse.

The device of the present invention includes an expandable anchor designed for transitioning from a linear configuration (collapsed) to a crown-like configuration (expanded). Such transitioning includes a radial expansion phase in which ends of the anchor curl out away from a center line (of the device) and an inversion phase in which ends of the anchor curl towards the center line. Such a transition between the collapsed and expanded states of the anchor ensures that when expanded in the atrium, the (free) ends of the anchor do not contact the inner walls of the atrium during any stage of expansion.

The device of the present invention further includes a transvalvular spacer attached to the distal end (opposite from delivery side) of the anchor. The spacer can be a solid spacer, a foam spacer or any other spacer that can provide a coaptation surface to valve leaflets (e.g., seal against the leaflets of a valve when closed). An embodiment of a spacer that includes a flexible column enclosed in an inflatable balloon is further described hereinbelow.

In order to enable controlled delivery of the device and maintain control over the anchor when expanded in the atrium, the present device further includes a ball-like graspable element at a proximal end. Such an element is graspable via a grasper and locking sleeve mechanism positioned at a distal end of a delivery catheter.

The ball like graspable element has multiple functions:
(i) it allows for loading of the device regardless of orientation;
(ii) when coupled with locking cup and O-ring the ball creates a fluid seal between the delivery system and implant of up to 2 Bar; and
(iii) The fluid seal is maintained regardless of the rotational position of the implant and up to a lateral tilt angle of up to 7.5 degrees.

These functions allow the anchor freedom to rotate while remaining axially attached to the delivery system and maintaining the fluid seal. This is of particular importance during delivery as the implant has to navigate bends in the DS which results in implant rotation. It also allows the anchor to rotate within the anatomy to a position of least energy during deployment.

Delivery of the device of the present invention is carried out using a dedicated catheter system (delivery system) having several unique features.

The catheter system includes three co-axial catheters each separately movable longitudinally. The distal end of the middle and outer catheters (also referred to herein as second and third catheters respectively) include the grasper (middle catheter) and locking sleeve (outer catheter) mechanism for grasping and releasing the anchor (and device). The distal end of the middle catheter also includes a seal for sealing against a fluid port positioned in the graspable element of the device. Such a seal enables filling and emptying of the balloon component of the spacer through a dedicated balloon valve that is actuated from the catheter system.

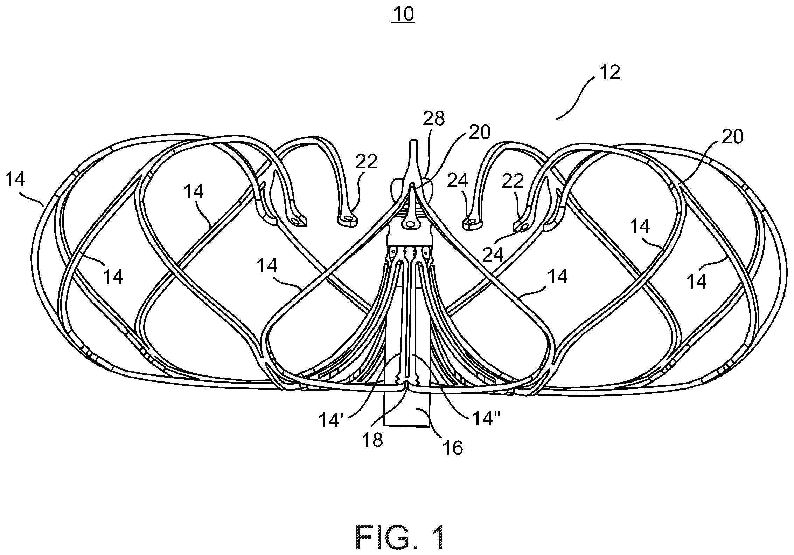

Referring now to the drawings, Figures 1-6 illustrate one embodiment of the present device which is referred to herein as device 10.

Figure 1 illustrates anchor 12 portion of device 10 in the expanded state. Anchor 12 includes struts 14 that are connected to a central column 16. In the embodiment shown in Figure 1, adjacent struts 14' and 14" of each strut pair (7 pairs shown) follow a parallel path away from central column 16 to diverge (branch) at 18 and converge (rejoin) at 20. Each strut pair ends in a spoon-like portion 22 that includes a pinhole 24. Pinhole 24 can be used as attachment points for controlled strut deployment. The spoon-like portion 22 of the crown tips is constructed specifically to prevent the leading edge of the crown tip from making contact with the inner lumen of the catheter when the implant is sheathed. The increased surface area of the crown tips (in comparison to the crown arms themselves) is to reduce the likelihood of the tip causing any damage to tissue either during or following deployment.

Struts 14 are fabricated from Nitinol or stainless steel by, for example laser cutting a tube or sheet. Struts 14 are 0.2-0.6 mm in width and 0.2-0.5 mm in thickness. Anchor 12 is 50-80 mm (e.g., 70.9 mm) in outer diameter (OD) and 15-30 mm (e.g., 20.7 mm) in height when expanded and 2-4 mm (e.g., 3.2 mm) in OD and 70-100 mm (e.g., 93.9 mm) in length when collapsed.

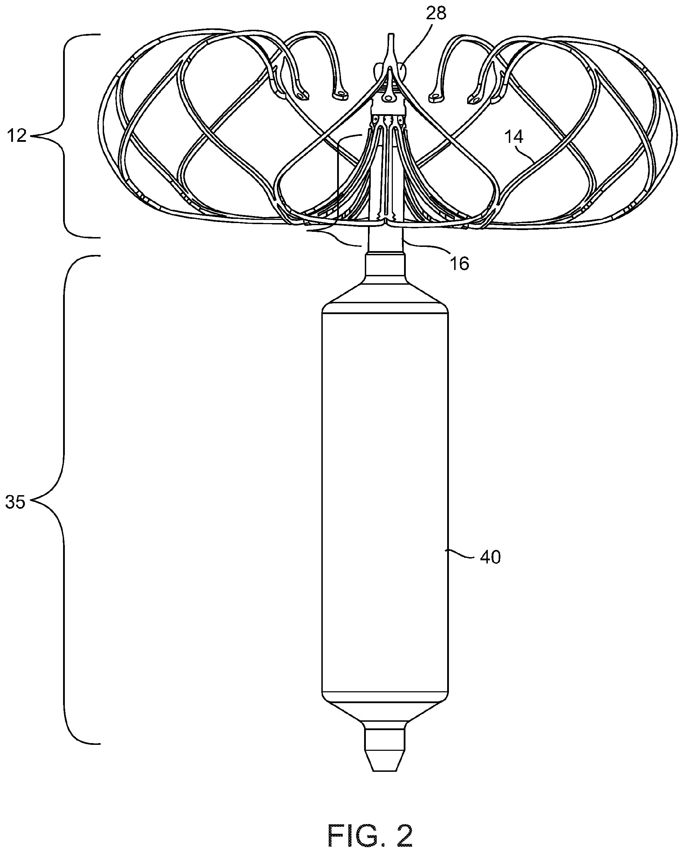

Figure 2 illustrates anchor 12 and attached balloon 40. Balloon 40 and column 16 are collectively referred to herein as spacer 35.

Struts 14 are pre-shaped such that when transitioning from a collapsed configuration (linearized within a delivery catheter) to a fully expanded configuration, the ends of struts 14 (e.g., spoon-like portion 22) do not contact the inner surface of the atrium.

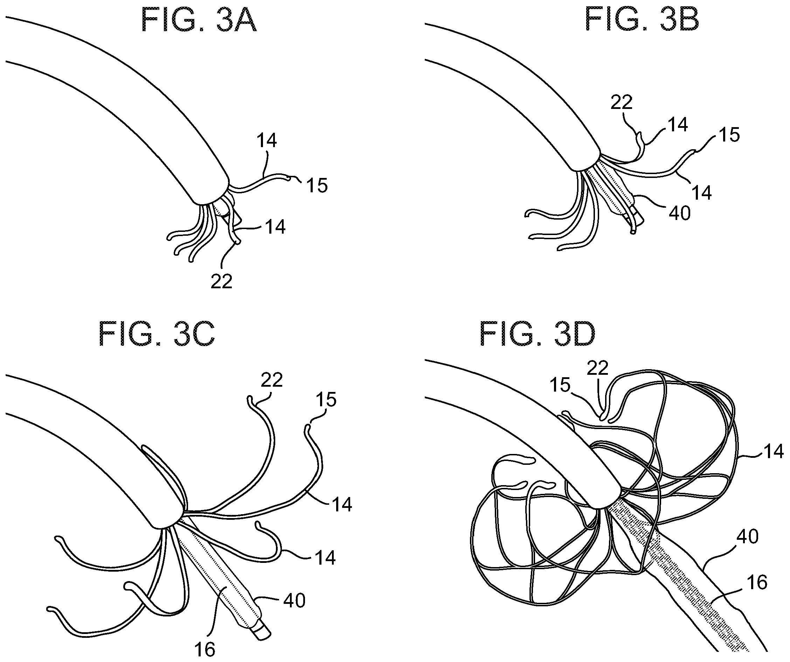

Figures 3A-D illustrate movement of struts and the ends thereof throughout expansion, spacer 35 and balloon 40 are also shown in these Figures. When anchor 12 first exits the delivery catheter, ends 15 of struts 14 move radially outward (away from the longitudinal centerline of device 10). At this point, the diameter of partially expanded anchor 12 is 10-20 mm which is far less than that of the atrial space in patients with moderate to severe valvular insufficiency. Figure 3B illustrates maximal diameter with ends 15 pointed outward (30-40 mm diameter), at this stage anchor dimeter is still less than that of the atrium. Further expansion (Figures 3C-D) sees ends 15 curl radially inward (towards centerline of device 10) to expand anchor to a final diameter of 60-80 mm without the risk of ends 15 contacting the inner walls of the atrium. In the expansion phases shown in Figures 3C-D, ends 15 follow a curved path that corresponds to the radius of curvature of the struts exiting the catheter tip.

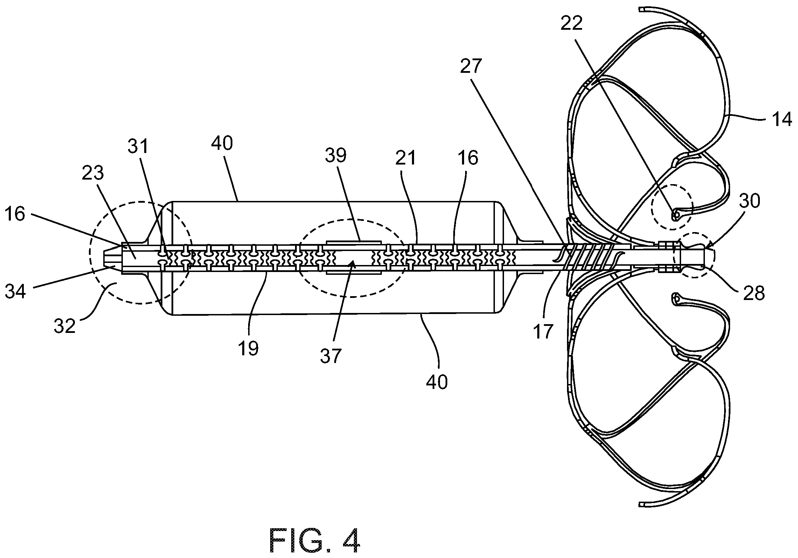

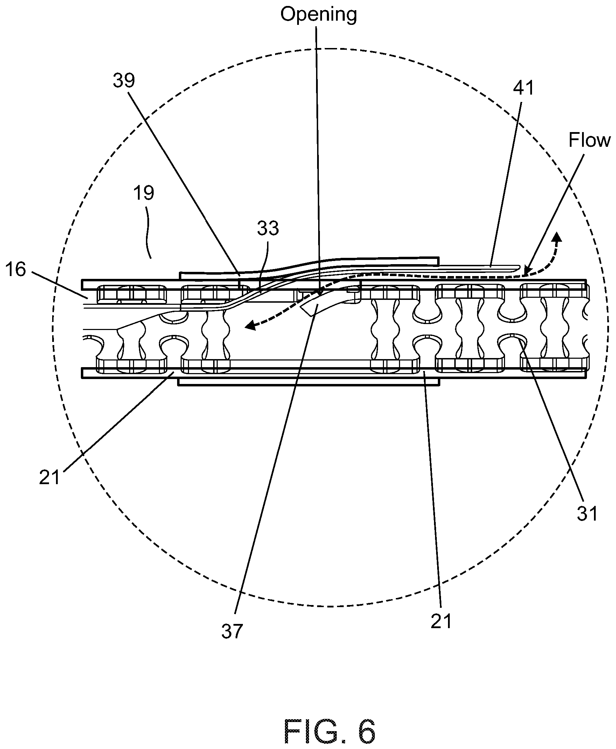

Central column 16 covers the length of device 10 and is configured with lateral flexibility (at a proximal portion 17 thereof) to provide a balloon 40 (Figure 2) attached thereto with tiltable (angle side-to-side, e.g., like a pendulum) flexibility. This enables a spacer 35 (central column 16 and balloon 40) to accommodate for asymmetrical leaflet closure and for off centerline anchor 12 placement. Central column 16 can be a polymer or Nitinol tube with sidewall cutouts 27 at proximal portion 17 (helical, double helix or a bow-tie shape). Central column 16 experiences cyclic axial loading throughout its life cycle due to the cyclic pressure differential between the ventricle and atrium the cutouts allow for flexibility of the central column during delivery but maintains sufficient axial rigidity to prevent buckling when under axial loading. Figure 4 illustrates a helical sidewall cutout pattern in proximal portion 17 of central column 16. Central column 16 can be 50-70 mm (e.g., 69.5 mm) in length and 2-4 mm OD x 1-3 mm ID (WT = 0.44 mm) in diameter. An inner lumen 23 (Figures 4 and 6) running the length of central column 16 can be 1-2 mm in diameter.

Central column 16 or a distal portion 19 thereof (portion surrounded by balloon 40, Figures 2 and 4) is covered with a polymeric (e.g., Carbothane 55D) sheath 21 (Figures 4 and 6) to which balloon 40 is bonded. Distal portion 32 of central column 16 can include a plug 34 for sealing around a guidewire.

Tube 31 includes an inward angled tab 37 for facilitating access to a valve 39 of balloon 40 (Figures 4 and 6). Valve 39 can be a silicone tube stretched over a portion of central column 16 that includes an opening into a volume of balloon 40. Valve 39 can be opened (forced away from the opening in central column 16) by guiding a valve actuating mechanism 33 (e.g., dedicated elongated element 41, Figure 6) through lumen 23 (e.g., from the delivery catheter) and under tab 37 (Figure 6). Once valve 39 is opened, balloon 40 can be filled through lumen 23 with the distal end plugged with plug 34. Plug 34 seals in the presence or absence of a guidewire running through lumen 23 and out therethrough.

Proximal end 26 of central column 16 includes a graspable element 28 for securing device 10 to a catheter system (further describe hereinbelow).

Graspable element 28 (best seen in Figure 4) is designed to be graspable within an actuatable grasper that includes at least two semi hemispherical halves that are configured to cup element 28. Element 28 is generally ball-shaped with a flat proximal end 30.

Graspable element 28 includes an opening at a proximal end 30 and thus also functions as a fluid port for filling balloon 40 of spacer 35 when device 10 is secured to the catheter system.

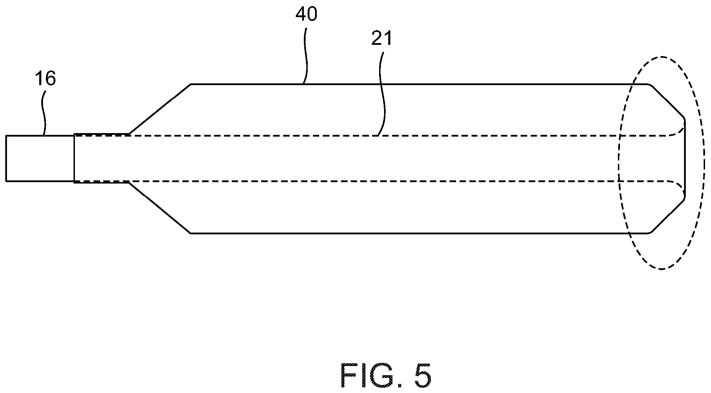

Balloon 40 can be bonded to sheath 21 with ends turned outward (as is shown in Figures 2 and 4) or alternatively, a distal end 44 of balloon 40 can be inverted inward and bonded to sheath 21 (Figure 5). Such a configuration ensures that potentially traumatic implant edges do not face tissue.

Balloon 40 is fabricated from a semi-compliant polymer using approaches well known in the art. Balloon 40 can be 42.0 ±1.5 mm in parallel length and 15 mm in diameter (when fully inflated). The pressure range for balloon 40 can be from 0 atmospheres (atm) to 0.25 atm within normal operation.

The wall of balloon 40 can be non-permeable or semi-permeable (e.g., permeable to a fluid such as water but not cells). A permeable balloon wall can be used for osmotic filling as is described herein below.

Balloon 40 can be filled with a fluid (e.g., saline or an osmotic solution) through the fluid port at graspable element 28. Filling through the port at graspable element 28 (through tube valve at 39) is described above and is further described hereinbelow with respect to the catheter system used for delivery of device 10.

Balloon 40 can be filled to a final volume and pressure following delivery of device 10 (i.e. following expansion of anchor 12). Due to the atraumatic nature of the anchorage system, the device can be fully deployed and the efficacy of the treatment can be assessed immediately. Should the device not result in a satisfactory reduction in regurgitation, the balloon can be deflated and the entire device retrieved into the sheath and removed from the patient.

The efficacy of balloon 40 in leaflet coaptation can be tested prior to device implantation. This can be achieved by simply using a similarly sized "off-the-shelf" balloon and advancing it into place along a guidewire across the valve. The clinician can then assess the efficacy of the spacer treatment before using the device. This has the potential to reduce the risk of treating patients who would not respond optimally to a spacer based valve repair.

As is mentioned hereinabove, balloon 40 can be semi-permeable to allow for osmotic filling. Balloon 40 can be partially inflated with an osmotic solution (e.g., saline contrast solution) having an osmotic potential that is greater than that of blood (e.g., osmolarity of 300-500 milliosmols/L).

The osmotic potential of blood is fixed. Therefore the osmotic potential of the solution used to fill balloon 40 will precisely determine the final pressure within the balloon. The osmosis mechanism ensures that the pressure with the balloon is consistent. In addition, the pressure that the balloon is filled with to intraoperatively does not need to be accurate since the osmotic mechanism will account for differences and will reach an equilibrium.

In the months and years leading up to the intervention, the patient's cardiovascular system compensates for deteriorating valve function. When the intervention is carried out, valve function is restored but the cardiovascular system is not accustomed to this change. Therefore, patients undergoing valve repair and/or replacement often suffer from symptoms due to pressure overloading. A potential treatment for this would be to underfill the balloon intraoperatively. In this manner the balloon would only partially improve valve function acutely. This would allow the cardiovascular system to remodel over the following weeks and months as the osmotic action of the balloon slowly increased the internal pressure/diameter which would further improve valve function.

As is mentioned hereinabove, device 10 of the present invention is configured for correcting incomplete leaflet coaptation in a heart valve. Device 10 can be used for correcting atrioventricular valve coaptation (bicuspid or tricuspid) by delivering device 10 using a catheter system. An example would be to treat the tricuspid valve. For this approach, the device would be delivered via a transcatheter approach either from the jugular vein through the superior vena cava or alternatively from the femoral vein and then into the inferior vena cava. In the case of the mitral valve, the preferred approach would be via a transseptal puncture to gain access to the left atrium and therefore the mitral valve.

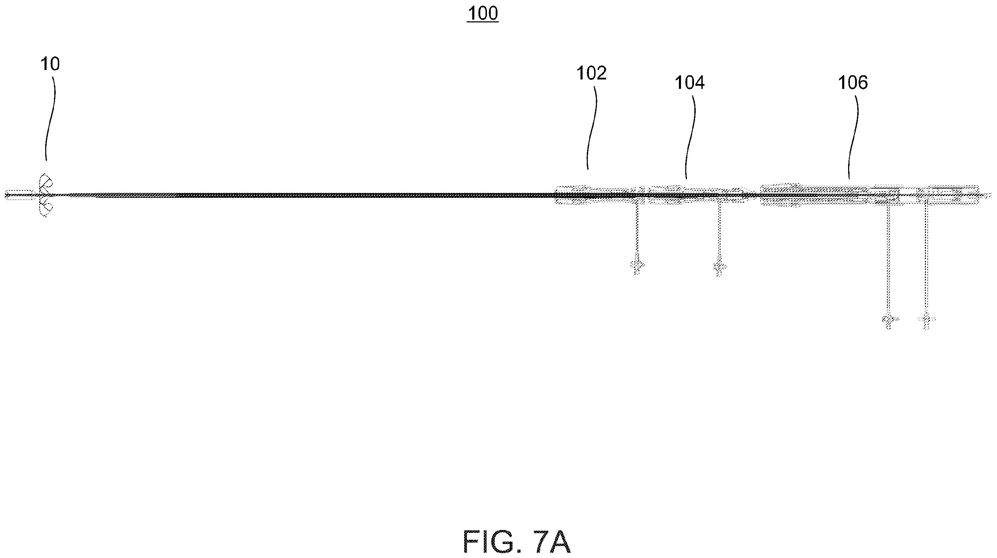

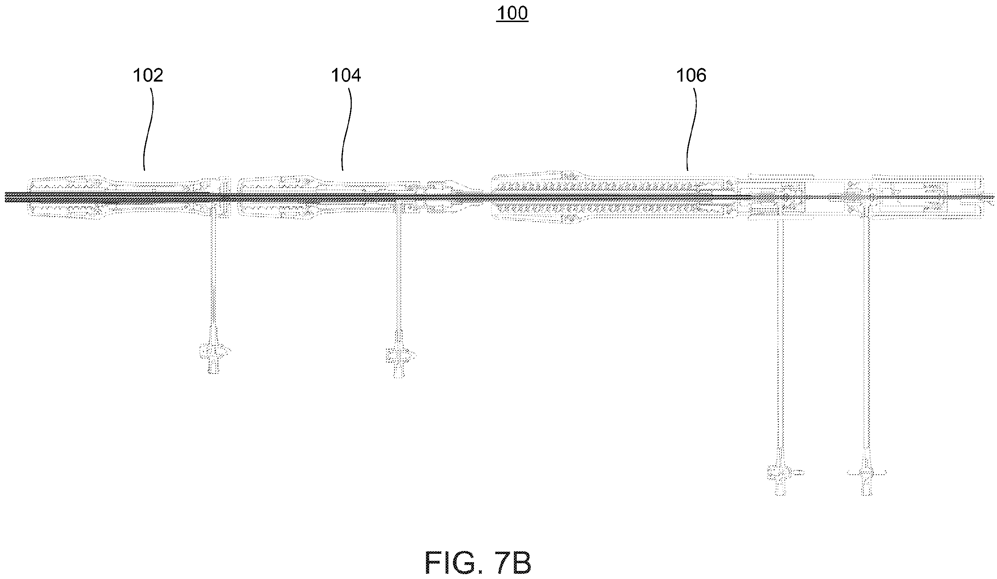

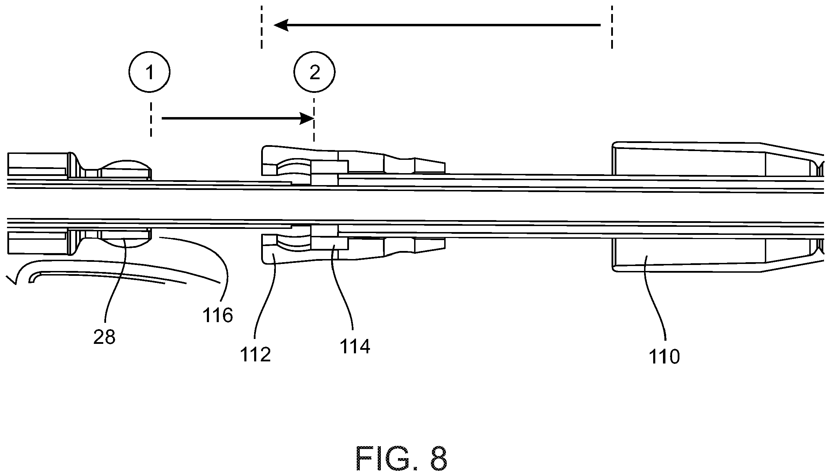

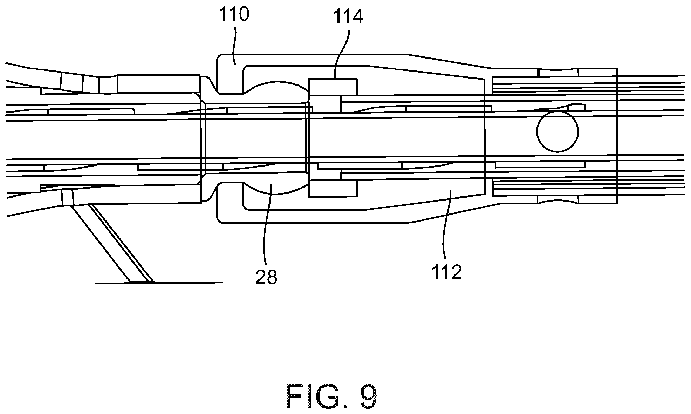

Figures 7A-9 illustrate one embodiment of a catheter system which is referred to herein as system 100.

Catheter system includes 3 coaxial catheters, an outer catheter 102, a mid-catheter 104 and an inner catheter 106.

Catheter 106 includes an elongation compensation mechanism to compensate for the elastic nature of the polymer tubes of the delivery system and the length of the polymer tubes. Because elasticity is determined by percentage, this elastic property can amount to a large change in absolute length at the distal end of catheter 106. The resultant changes in length can cause relative motion between components at the distal end which can be compensated for by spring loading a part of the system.

The compensation mechanism includes two parts, locking cup 110 and grasper 112, which act together to grip graspable element 28 of device 10. As catheter 106 can be under large tensile loads during implant deployment and retrieval, a compression spring (in the handle) is used to allow locking cup 110 to maintain its position relative to grasper 112. In this manner locking cup 110 and grasper 112 stay locked in the closed position at the distal end of catheter 106 and graspable element 28 of device 10 remains securely attached within locking cup 110. An O-ring 114 positioned within grasper 112 maintains a seal against a front face 116 of graspable element 28.

Catheter system 100 is used to deliver device 10 as follows. Prior to delivery, system 100 is prepared on a benchtop sterile area by laying out system 100 components and accessories and testing each as well as the overall system. Device 10 is removed from packaging and loaded onto catheter system via graspable element 28 and pulled into inner steerable catheter 104. Inner steerable catheter 104 with sheathed implant 10 can then be loaded into outer catheter 102.

In an exemplary trans-septal approach, a jugular vein access is created and an 18F introducer is positioned through the jugular vein access. A femoral vein access is created and a 6F introducer is placed therethrough. A Trans Esophageal Echo (TEE) or Trans Thoracic Echo (TTE) probe is then placed according to standard procedure to assess tricuspid valve functionality. Under fluoroscopy, a compliant 1.5 ml balloon catheter is inserted through the 18F introducer and advanced to the right atrium, through the tricuspid valve and right ventricle and into the pulmonary artery. A 0.89mm (0.035") guidewire is inserted through the balloon catheter into the pulmonary artery and the balloon catheter is retrieved while holding wire in place. A contrast agent is injected though the 6F introducer to identify chambers outline and the tricuspid valve level. Under fluoroscopy, a 15 mm diameter off-the-shelf balloon catheter is threaded over the wire until the balloon is positioned across the tricuspid valve. The balloon is inflated and the regurgitant flow within the valve is assessed under echocardiography. If a satisfactory reduction in regurgitation is evident, the balloon is retrieved and the procedure is continued, otherwise the balloon is retrieved and the procedure is aborted.

The proximal tip of the guidewire is inserted through device 10 and catheter system 100. While maintaining the guidewire stationary relative to the anatomy, system 100 is advanced until at approximately the level of mid atrium. The steerable catheters 102 & 104 can be used to correctly orientate the system relative to the patients anatomy. Once satisfactorily positioned, the tips of anchor 12 are expanded into the right atrium under fluoroscopic guidance in a controlled fashion and under continuous echocardiography assessment of valve functionality. Once anchor 12 is deployed, leaflet motion and device 10 position is assessed under echocardiography and/or fluoroscopy using contrast agent injection through the 6F catheter. If the tricuspid valve shows typical motion the procedure continues otherwise the tips of anchor 12 are retracted and repositioned and deployment is repeated.

A 20-50 ml syringe filled with (10 - 50%) 20% saline/sterile water contrast media solution is attached to the inflation valve/port of balloon 40, the guidewire is retracted just enough to leave the distal plug sealed and balloon 40 is then filled under fluoroscopy. As balloon 40 approaches its fill volume, cyclic indentations due to the closing valve leaflets should be clearly visible and the balloon is further inflated until the leaflets no longer cause indentations on the balloon and it has reached its nominal diameter. Catheters 102 and 104 are then retracted such that catheter 106 is fully extended to allow maximum implant movement in-situ. Echocardiography is then used to verify the position of balloon 40 and efficacy in reducing regurgitation.

If balloon 40 is not efficient in reducing regurgitation, device 10 is collected and system 100 and attached device 10 are removed. If the position of device 10 is not satisfactory, balloon 40 can be deflated via vacuum and device 10 can be repositioned.

If device 10 position and function is satisfactory, balloon 40 diameter and shape are monitored under fluoroscopy while unlocking and retracting the valve actuating mechanism to seal the spacer balloon. Device 10 can then be released from catheter system 100 and the guidewire and catheter system 100 are then removed.

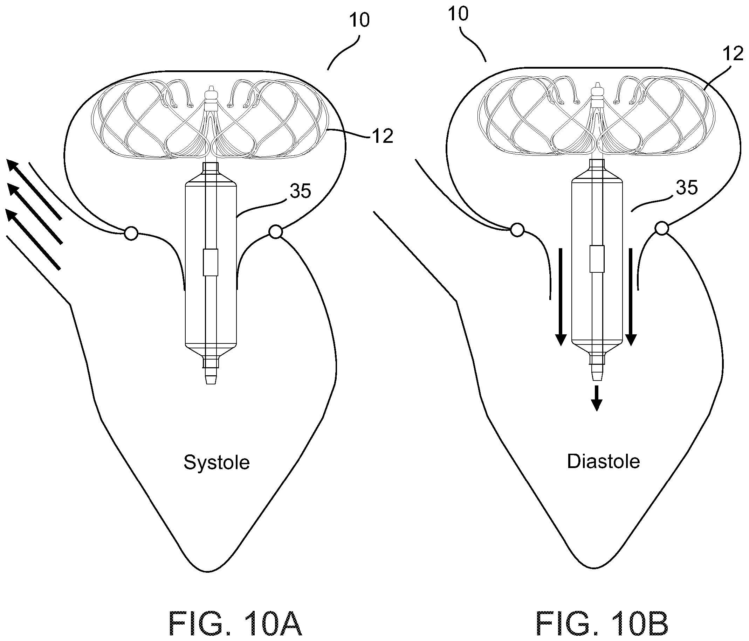

Once anchor 12 is positioned in an atrium and balloon 40 is inflated, spacer 35 of device 10 seals against the partially closed valve leaflets during systole (Figure 10A) and enables blood flow through the valve during diastole (Figure 10B) when the leaflets are fully open.

As used herein the term "about" refers to ± 10 %.

Additional objects, advantages, and novel features of the present invention will become apparent to one ordinarily skilled in the art upon examination of the following examples, which are not intended to be limiting.

### EXAMPLES

Reference is now made to the following example, which together with the above descriptions, illustrate the invention in a non-limiting fashion.

### Animal Studies

Animal studies were conducted in order to evaluate the clinical safety and performance of the present device and delivery system in a healthy swine model in a chronic setting. The device was implanted in 6 animals with follow-up periods of 90 days.

The objectives of the animal studies were to evaluate safety and sizing of the device.

Implant delivery and placement was performed in each of the animals using the steps described hereinabove. Histopathological results and long term clinical results were used to demonstrate the safety of the system.

The 6 animals were monitored via angiographic imaging at 1 week, 1 month, 2 month and 3 month post procedure. The animals were sacrificed following the last monitoring stage. Thorough evaluation of the treated animals revealed no clinically significant device related effects. The implant was intact and well positioned.

### Histology analysis results

Post animal sacrifice the heart was explanted and sent to histology analysis to evaluate safety parameters such as structural impairment, tissue growth, thromboses and the like.

Analysis revealed no structural impairment of heart tissues. Valve leaflets were intact as well as ventricular walls. There was no interference with papillary muscle or chordae. Smooth muscular tissue growth was observed over crown arms and no thrombus formation was evident.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

In addition, any priority document(s) of this application is/are hereby incorporated herein by reference in its/their entirety.

## Claims

1. A device for treating valvular regurgitation, comprising:
an expandable anchor comprising a plurality of struts configured to transition from a first configuration when trapped within a tube to a second configuration when released from the tube, the transition comprising an expansion phase in which a plurality of ends of the plurality of struts curl out and an inversion phase in which the plurality of ends of the plurality of struts curl in; and
a spacer configured for providing a coaptation surface to at least one valve leaflet and being attached to the expandable anchor, the spacer comprising a column enclosed in an inflatable balloon.

2. The device of claim 1, wherein the expansion phase in which the plurality of ends of the plurality of struts curl out away from a center line of the second configuration.

3. The device of claim 2, wherein the inversion phase in which the plurality of ends of the plurality of struts curl in the center line of a crown-like configuration.

4. The device of claim 1, wherein the first configuration is a linear configuration, and wherein the second configuration is a crown-like configuration.

5. The device of claim 1, wherein the column comprises a tab configured to facilitate access to a valve, the tab being angled inward relative to the column.

6. The device of claim 1, wherein the column is covered with a polymer sheath, the polymer sheath being bonded to the inflatable balloon at a portion of the column.

7. The device of claim 6, wherein the inflatable balloon is bonded to the polymer sheath, the inflatable balloon comprising a configuration with at least one end turned outward or inverted inward.

8. The device of claim 6, wherein the inflatable balloon comprises an inverted end, the inverted end being bonded to the polymer sheath.

9. The device of claim **1,** wherein the column comprises an inner lumen extending along a length of the column, the inner lumen being configured to receive a valve actuating mechanism or a guidewire.

10. The device of claim **1,** wherein the column comprises at least one cutout formed along a proximal portion of the column, the at least one cutout being configured to provide lateral flexibility.

11. The device of claim **1,** wherein the column comprises a distal portion that comprises a plug configured for sealing around a guidewire.

12. The device of claim **1,** wherein the expandable anchor comprises seven struts, each of the seven struts branching and rejoining along a length thereof.

13. The device of claim **1,** wherein the expandable anchor comprises struts that comprise a spoon-like portion at an end thereof, the spoon-like portion comprising a pinhole.

14. The device of claim **1,** wherein the expandable anchor comprises struts that comprise an eyelet at an end thereof, the eyelet being configured for use as an attachment point.
